# EUROPEAN PATENT SPECIFICATION

(11) **EP 2 814 319 B1**
(45) Date of publication and mention of the grant of the patent: **14.11.2018**
(21) Application number: 12706017.6
(22) Date of filing: 17.02.2012
(51) Int. Cl.: A01N 25/30, A01N 33/12, A01N 47/44, A01N 55/08, A01N 59/16, A61K 33/38, A01P 1/00

(54) **ANTIMICROBIAL COMPOSITION CONTAINING PHOTOCHEMICALLY STABLE SILVER COMPLEXES**
ANTIMIKROBIELLE ZUSAMMENSETZUNG MIT FOTOCHEMISCH STABILEN SILBERKOMPLEXEN
COMPOSITION ANTIMICROBIENNE CONTENANT DES COMPLEXES D'ARGENT PHOTOCHIMIQUEMENT STABLES

(43) Date of publication of application: 24.12.2014
(73) Proprietor: NM Tech Nanomaterials and Microdevices Technology Ltd., London W1S 4LR (GB)
(72) Inventor: DISSETTE, Valeria, I-44121 Ferrara (IT); CARLI, Stefano, I-44123 Ferrara (IT); PAZZI, Daniele, I-44123 Ferrara (IT); BIGNOZZI, Carlo Alberto, I-44121 Ferrara (IT)
(74) Representative: Long, Giorgio
(86) International application number: PCT/EP2012/052755
(87) International publication number: WO 2013/120532

(56) References cited:
- EP-A1- 0 179 583
- EP-A1- 2 157 211
- WO-A1-2007/122651
- US-A- 5 057 518
- US-A- 5 616 338
- US-A1- 2003 035 848

## Description

### Field of the invention

The present invention relates to an antimicrobial composition containing photochemically stable silver complexes and micelle adducts.

### Background art

The use of topical antimicrobial agents for wound care gained a wide acceptance in the 1960s once it was discovered that treating burns with silver nitrate decreased the number of deaths that were a result of sepsis from 60% to 28%.

Antiseptic silver sulfadiazine (SSD) was associated with additional decreases in infection, eventually making a place for itself in general wound care. Silver sulfadiazine demonstrated improved outcomes and decreased infection rates.

Antiseptics differ from antibiotics in that they have broad-spectrum activity and can be effective against many types of organisms including aerobic and anaerobic bacteria, yeasts, fungi, and molds.

Bacterial resistance to antibiotics is extensively documented in medical literature. Resistance to antiseptics, however, has only been studied more recently. Some authors compared the ability of Staphylococcus epidermidis to develop resistance to various antibiotics and antiseptics. Results suggested that the bacteria developed resistance to the antibiotics minocycline and rifampicin, however no evidence of resistance was observed with chlorhexidine, silver sulfadiazine and polyhexametylene biguanide.

Silver has been used as an antimicrobial agent for thousands of years. Silver ions exert varying antimicrobial effects depending on their binding site. When binding occurs at the bacterial cell wall, ruptures can occur. When bound to proteins involved in respiration and nutrition of the organism, silver blocks these processes and the bacterium dies. When binding to DNA, silver can affect the replication and division of the organism. The activity of silver lies in its ionic form. Silver is an effective antimicrobial agent with low toxicity, which is important especially in the treatment of burn wounds where transient bacteremia is prevalent and its fast control is essential. It has been proved that the monovalent Ag⁺ ion shows a rather broad spectrum of bactericidal activity.

Elemental silver and silver salts demonstrate substantially less effectiveness against microbes. Previously, silver salt solutions, such as silver nitrate, were used to bathe the wound. These required large amounts of silver to achieve the desired effect. Silver sulfadiazine (SSD) creams enable much lower amounts of silver to be effective and act by discharging silver ions when in contact with wound exudate. The silver ions, however, may be rapidly neutralized and require daily or more frequent application of SSD. The amount of silver released into the wound is not always clearly defined and can be a concern for toxicity in healthy tissue.

Ag⁺ -based formulations are thus applied in topical use pharmaceutical preparations as well as in the formulation of disinfectants for environmental applications.

Formulations based on Silver salts, or on Silver Sulphadiazine, suffer in general of photochemical instability and, in the latter case, of low water solubility.

Colloidal Silver nanoparticles have been the focus of increasing interest and are being heralded as an excellent candidate for therapeutic purposes. The term "colloidal" refers to a substance that consists of ultra-fine particles that do not dissolve, but remain suspended in a medium of different matter. These ultra-fine particles are too small to be seen by the naked eye. The silver particles are electronically charged to activate the germicidal quality of the silver and allow the particles to remain suspended in the solution of deionized water. The colloidal preparation is however photochemically unstable, in fact visible light causes the photoreduction of Ag⁺ to Ag° with a consequent decrease of the antimicrobial action. The production of Ag⁺ ions from metallic Silver Ag°, is in addition limited by the high oxidation potentials of Ag° to Ag⁺ (E1/2 = + 0.80 V vs normal Hydrogen Electrode, at 25°C) which implies an equilibrium constant for silver oxidation in the order of 1.2 x 10⁻⁶, indicating that 1 M of Ag° (107.8 g) can produce, at the equilibrium, only 1 x 10⁻⁴ g of Ag⁺.

Considering that Ag⁺ is active against over 600 different types of Bacteria, Fungi and Viruses the search for newer and superior in stability silver-based antimicrobial agents is obviously of interest.

In a previous patent application, published under WO 2007/122651, we reported on the preparation of novel photochemically stable antibacterial and antiviral nanomaterials based on metal oxides or metalloid oxides, such as, e.g., TiO₂, ZrO₂, SnO₂, ZnO, and SiO₂, functionalized with molecular species of organic nature, capable of binding simultaneously to the oxide and to ions of transition metals, such as Ag⁺.

### Summary of the invention

The present invention relates to Silver complexes and micelle systems of cationic surfactants trapping a neutral Silver complex which are thermally and photochemically stable in aqueous solution and exhibit a strong antimicrobial activity.

An object of this invention is a micelle adduct of a photochemically stable neutral complex of monovalent Silver of formula **Ag-L**, wherein L is 4-mercaptophenylboronic acid, as defined in the appended claims.

Another object of the invention is a antimicrobic formulation containing a micelle system of the complex of formula **Ag-L**.

A further object of the invention is a process for producing the complex of formula **Ag-L** and its micelle systems.

A still further object is constituted by the micelle system of the complex of formula **Ag-L** or of the formulation containing it for use in medicine, particularly as antimicrobic agent.

A still further object is the non-therapeutical use of the micelle system of the complex of formula **Ag-L** or of a formulation containing it as a disinfectant, in particular for the treatment of surfaces of objects.

### Brief description of the drawings

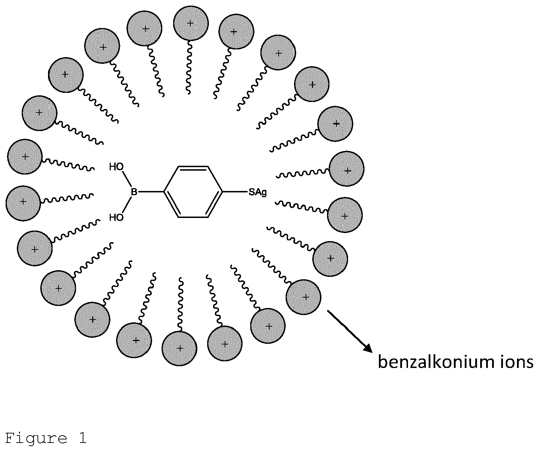

Figure 1 shows a schematic view of the micelle structure formed from Benzalkonium ions with the hydrophobic tails trapping the neutral **Ag-L** complex of the invention.

### Detailed description of the invention

The invention relates to a micelle adduct of a neutral complex of monovalent Silver of formula **Ag-L**, wherein Ag is an Ag⁺ ion and L is a ligand of formula 4-mercaptophenylboronic acid, and wherein the silver ion is linked to the mercapto group of the ligand L.

4-Mercaptophenylboronic acid has the following structure:

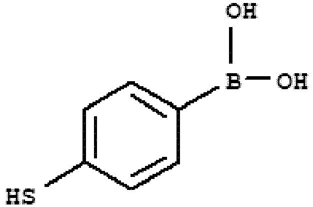

The complex of formula **Ag-L** can be prepared by adding stoichiometric amounts of 4-mercaptophenylboronic acid to an aqueous solution of a silver salt, such as silver nitrate. This addition results in the formation of a **Ag-L** neutral complex, which is only partly soluble in water. This complex can be solubilized in aqueous solution by addition of a cationic surfactant.

In a preferred embodiment, the cationic surfactant is a quaternary ammonium salt. More preferably, the surfactant is selected from a group consisting of Benzyl(dodecyl)dimethylammonium chloride, Benzyl(tetradecyl)dimethylammonium chloride, Benzyl(hexadecyl)dimethylammonium chloride, Benzalkonium chloride (which is the mixture of the three substances above) and mixtures thereof.

In another preferred embodiment the surfactant is Chlorexidine or Polyhexamethylene Biguanide cations (**PHMB**). PHMB has the following structure:

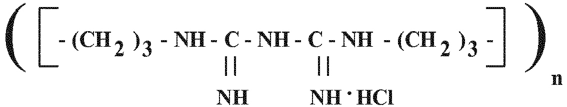

PHMB cations are formed upon dissociation in water of the chloride ion.

If a quaternary ammonium salt is used, the concentration of this surfactant in the water solution is about 10⁻² M or higher.

If chlorexidine or PHMB are used as a surfactant, their concentration in the aqueous solution is about 0,6-1 %w/v.

It has been found that the use of a surfactant does not only solubilize di **Ag-L** complex, but it also provide for a stabilization of the neutral complex. Both solubilization and stabilization occur when the surfactant is use at a concentration that is critical for the formation of micelles.

This fact is consistent with a stabilizing effect due to trapping of the neutral **Ag-L** complex inside the micelle formed by the surfactant as shown schematically in Figure 1 (for benzalkonium chloride).

The observation that, after a 12 month period, in the presence of a cationic surfactant no precipitation and no formation of metallic Silver occur, confirms the relevant role of the cationic surfactants in determining the stabilization in solution of the neutral complex and the role of the ligand L in stabilizing photochemically the Ag⁺ ion.

The present invention thus relates to a micelle adduct of a neutral complex of formula **Ag-L** as depicted above in a micelle system formed by a cationic surfactant. The cationic surfactant is preferably selected from the group consisting of Benzyl(dodecyl)dimethylammonium chloride, Benzyl(tetradecyl)dimethylammonium chloride, Benzyl(hexadecyl)dimethylammonium chloride, Benzalkonium chloride, chlorexidine, Polyhexamethylene Biguanide and mixtures thereof.

### EXAMPLE

The neutral complex **Ag-L** with L = 4-mercaptophenilboronic acid was prepared by adding stoichiometric amounts of 4-mercaptophenylboronic acid to an aqueous solution of a silver salt, such as silver nitrate. This addition results in the formation of a **Ag-L** neutral complex, which is only partly soluble in water. The aqueous solution of the complex was stabilized by addition of Benzalkonium chloride up to reach a concentration of about 2 x 10⁻² M, which corresponds to the double of its critical micelle concentration. Aqueous solutions of **Ag-L** did not show any change in color or composition after 12 months to ambient light exposure. In comparable conditions solutions of AgNO₃ showed formation of a black Ag° precipitate after few minutes.

### Chemical characterization

### Elemental analysis of Ag-L

Calculated for AgSC₆H₆BO₂: C, 12.29; H, 2.32; S, 12.29
Found: C, 12.28; H, 2.30; S, 12.28

In addition to elemental analysis data, which are fully consistent with the formula of the neutral complex, infrared spectroscopy data confirm the coordination of the Silver ion to the Sulfur atom of the organic ligand. The uncoordinated free ligand, in fact, exhibits IR band at 2663 cm⁻¹ which can be confidently assigned to the corresponding S-H stretching mode. Upon coordination of Silver ions and binding of Silver to the Sulfur end of the ligand, such a band disappears leaving unmodified the other parts of the IR spectrum. The disappearance of the S-H stretching band confirms that the binding of Silver ions to Sulfur has occurred. According to Sandroff et al. J. Phys Chem. 1992, 86, 3277*,* the S-Ag stretching bands should be expected in the frequency range 150-250 cm⁻¹ which is however beyond the instrumental limit of the used IR spectrometer (4000-400 cm⁻¹)

### Determination of the micelle size

Determination of the micelle size was performed with a Malvern Instrument ZETASIZER 3000, at 25°C with a detector angle of 90°, on the following aqueous solutions containing respectively: **1)** benzalkonium chloride **(Bz)** (0.6% v/v); **2) PHMB** (1% v/v); **3) AgL- Bz** (Ag-L, 3.3 x 10⁻⁴ M; Bz 0.6% v/v); **4) AgL- PHMB** (Ag-L, 3.3 x 10⁻⁴ M; PHMB, 1% v/v); **5) AgL- BZ- PHMB** (Ag-L, 3.3 x 10⁻⁴M; BZ, 0.3% v/v; PHMB, 0.5 % v/v).

| Table I. Size report on the average dimensions (Z Average Mean) and Z Potential of the micelle formed in aqueous solutions containing different cationic surfactants and mixtures of cationic surfactants and of the **AgL** complex at constant concentration = 3.3 x 10⁻⁴ M. | | |
|---|---|---|
| Solution compositions (concentrations in water) | *Z Average Mean* (nm) | *Z Potential* |
| **AgL- Bz** (Ag-L, 3.3 x 10⁻⁴ M; Bz 0.6% v/v) | **104** | **40.5** |
| **Bz** (0.6% v/v) | **32** | **13.3** |
| **AgL- PHMB** (Ag-L, 3.3 x 10⁻⁴ M; PHMB, 1% v/v) | **192** | **32.5** |
| **PHMB** (1% v/v) | **8** | **0.6** |
| **AgL- BZ- PHMB** (Ag-L, 3.3 x 10⁻⁴M; BZ, 0.3% v/v; PHMB, 0.5 % v/v) | **139** | **33** |

Formation of the micelle adducts is confirmed by the size reported in Table I.

Solutions containing only the cationic surfactants Bz or PHMB form micelle with average diameters of 32 nm and 8 nm and with a Z potential of 13.3 and 0.6 respectively. In presence of **AgL** molecules, the micelles formed by the cationic surfactants at the same concentration level undergo to an increase in size: **AgL-Bz** increases up to 104 nm while for **AgL- PHMB** the average dimension goes up to 192 nm, suggesting trapping of an higher number of **AgL** species. According to the increase in micelle size also the Z potential increases to 40.5 for **AgL- Bz** and to 32.5 for **AgL- PHMB**.

### Bactericidal Activity

The bactericidal activity was tested on freshly prepared aqueous solutions of the neutral **Ag-L** complex where the ligand **L** coordinated to the silver ion is 4-mercaptophenilboronic acid .

Additional tests were carried out on aqueous solutions containing the neutral Silver **Ag-L** complex and different cationic surfactants and, for purpose of comparison, on aqueous solutions containing only the cationic surfactants at the same concentration levels. It is in fact known that the cationic surfactants used for the purposes of the invention are endowed with antimicrobic activity on their own.

The bactericidal activity tests were carried out according to UNI EN 1272:2009 and UNI EN 13697:2001 standards, which make reference to chemical disinfectants and antiseptics, by using the following test organism: *Staphylococcus aureus* (ATCC- 6538), as representative of Gram-positive bacteria and *Pseudomonas aeruginosa* (ATCC - 15442), as representative of Gram-negative bacteria.

According to UNI EN 1272:2009, which is a quantitative **suspension** test for the evaluation of bactericidal activity, it was found that **Ag-L** complex (**L** = 4-mercaptophenilboronic acid) in concentration of the order of 3.3 x 10⁻⁴ M in water, was able to cause a 2-3 log reduction of bacterial count after 30 min of contact, analogously to what observed for a Silver Nitrate solution of comparable concentration, which however was observed to lose the antimicrobial activity after exposure to ambient light, due to reduction of Ag⁺ to Ag°. Analogous tests, performed on aqueous solutions containing mixtures of the Silver complex **AgL** with the cationic surfactants Bz (0.6 %v/v) or PHMB (1% v/v) or on aqueous solutions containing mixtures of the Silver complex **AgL** with both cationic surfactants Bz (0.3 %v/v) and PHMB (0.5 v/v) gave after 5 minutes of contact a very strong reduction of the bacterial count of the order of 8 log, which was superior to that observed on solutions containing only the cationic surfactants at the same concentration level. On such solutions it was in fact observed a maximum reduction of the bacterial count of ca 6-7 log.

Additional tests carried out in dirty condition, in presence of bovine serum albumin, which is known to deactivate the antimicrobial action of quaternary ammonium salts, revealed a very strong bactericidal activity between the Ag+ cation contained in the **AgL** complex and the cationic surfactants. The test was performed according to UNI EN 13697:2001, which is a quantitative non-porous **surface** test for the evaluation of bactericidal activity in dirty conditions (3.0 g of bovine serum albumin per liter of water). The results of these tests are summarized in Table II

| Table II. Bactericidal activity of sample aqueous solutions containing different chemical species, which include: Silver complexes, mixtures of Silver complexes and cationic surfactants and cationic surfactants, evaluated according to UNI EN 13697:2001, in dirty conditions, allowing a time contact with the microorganism of 5 minutes. | | |
|---|---|---|
| Chemical species (concentrations in water) | *Staphylococcus aureus* (log reduction) | *Pseudomonas aeruginosa* (log reduction) |
| **AgL** (3.3 x 10⁻⁴ M) | **< 1** | **< 1** |
| **AgL- Bz** (Ag-L, 3.3 x 10⁻⁴ M; Bz 0.6% v/v) | **4** | **4** |
| **Bz** (0.6% v/v) | **< 1** | **< 1** |
| **AgL- PHMB** (Ag-L, 3.3 x 10⁻⁴ M; PHMB, 1% v/v) | **5** | **5** |
| **PHMB** (1% v/v) | **2** | **2** |
| **AgL- BZ- PHMB** (Ag-L, 3.3 x 10⁻⁴M; BZ, 0.3% v/v; PHMB, 0.5 % v/v) | **> 5** | **> 5** |
| **BZ-PHMB** (BZ, 0.3% v/v; PHMB, 0.5 % v/v) | **2** | **2** |

The reduction of bacterial count observed indicate that in dirty conditions, while Bz and the Ag+ complexes alone are substantially inactive and PHMB shows only a modest activity, the association between the **AgL** complex with the cationic surfactants Bz or PHMB allow for a remarkable bactericidal effect which is maximized in the case of the solution containing both cationic surfactants, **AgL- Bz- PHMB.**

The amplification of the bactericidal effect through association of **AgL** and **Bz** or **PHMB** ions is consistent with a synergic effect between the silver ion and the cationic surfactant. The latter is thought to decrease intermolecular interactions causing the dissociation of the cellular membrane lipid bilayers. Silver ions can thus bind easier functional groups such as thiol (-SH), carboxylic (-COOH) or amino (NH₂) groups, present in the membrane cell proteins, causing the killing of the bacterial species. It should be noted in addition that bacteria are negatively charged species: as a consequence, association with the positively charged micelle structure, schematized in Figure 1, of the cationic surfactant trapping the neutral **AgL** complex, should be favored.

An object of the invention are the micelle adducts as defined above for use in medicine, particularly as antimicrobic agents.

Preferably, the micelle adducts of the invention are active against the following bacteria:
*Legionella pneumophila, Pseudomonas aeruginosa, Staphilococcus aureus, Enterococcus faecalis, Escherichia coli, Salmonella enteridis D1, Listeria monocytogenes, Neisseria gonorrhoeae, Neisseria meningitidis,Vibrio cholerae, MRSA, Clostridium Diff, Acinetobatcter Baumanii, Mycobacterium terrae, Mycobacterium avium and Bacillus subtilis;* Fungi such as *Candida albicans and Aspergillus niger;* and with the following Viruses: A*H1N1, Adenovirus, Poliovirus, Citomegalovirus, Enterovirus, Herpes virus, Hepatitis A Virus, Hepatitis B Virus, Human Immunodeficiency virus (HIV)*, *Varicella Zoster Virus, Aviaria Viruses Group and SARS Corona Virus.*

A pharmaceutical composition containing an antimicrobic amount of a micelle adduct as defined above together with pharmaceutically acceptable eccipients is another object of the invention.

Another object of the invention is a pharmaceutical composition of a micelle adduct of a complex of formula **Ag-L** as defined above with a cationic surfactant.

This composition will be preferably a liquid or semi-liquid composition, such as solutions, gels, suspensions, lotions, ointments, etc..

The pharmaceutical compositions of the invention can be prepared according to conventional methods, such as the ones described in Remington's Pharmaceutical Sciences Handbook, Mack Pub. Co., N.Y., USA, 17th edition, 1985.

Another object of the invention is a non-therapeutical formulation, particularly a liquid formulation such as a solution, containing a micelle adduct of a neutral complex of formula **Ag-L** in an antimicrobic amount with a cationic surfactant.

The non-therapeutical formulations of the invention can be used as disinfectants, particularly for the treatment of the surfaces of objects of various nature. As an example, the non-therapeutical formulation of the invention can be used to disinfect floors, walls, furniture, apparatuses, devices, particularly in a medical environment such as an hospital or the like. They can also be used to disinfect the hands of an operator.

## Claims

1. A micelle adduct of a neutral complex of monovalent Silver of formula **Ag-L** wherein Ag is an Ag⁺ ion and L is a ligand of formula 4-mercaptophenylboronic acid, and wherein the silver ion is linked to the mercapto group of the ligand L in a micelle system formed by a cationic surfactant.

2. The micelle adduct of claim 1, wherein the cationic surfactant is a quaternary ammonium salt.

3. The micelle adduct of claim 1, wherein the cationic surfactant is selected from the group consisting of Benzyl(dodecyl)dimethylammonium chloride, Benzyl(tetradecyl)dimethylammonium chloride, Benzyl(hexadecyl)dimethylammonium chloride, Benzalkonium chloride, Chlorexidine, Polyhexamethylene Biguanide and mixtures thereof.

4. A process for preparing the product according to any of claims 1 to 3, comprising the following steps:
- adding stoichiometric amounts of 4-mercaptophenylboronic acid to an aqueous solution of a silver salt to form the complex **Ag-L** of claim 1,
- adding a cationic surfactant to form the micelle adduct of any claim 2 to 4.

5. The process of claim 4, wherein the silver salt is silver nitrate.

6. The process of claim 4 or 5, wherein, if a quaternary ammonium salt is used as the surfactant, the concentration of this surfactant in the water solution is about 10⁻² M or higher.

7. The process of claim 4 or 5, wherein, if chlorexidine or Polyhexamethylene Biguanide are used as the surfactant, the concentration of this surfactant in the aqueous solution is about 0,6-1 %w/v.

8. The product of any of claims 1 to 3 for use in medicine.

9. The product of any of claims 1 to 3 for use as antimicrobic agents.

10. A pharmaceutical composition containing an antimicrobic amount of the product of any of claims 1 to 3 together with pharmaceutically acceptable eccipients.

11. A non-therapeutical liquid formulation containing the product according to any of claims 1 to 3.

12. The non-therapeutical use of the product according to any of claims 1 to 3 as a disinfectant.

13. The non-therapeutical use of claim 12, for the treatment of surfaces of objects.

14. The non-therapeutical use of claim 13, wherein said surfaces are floors, walls, furniture, apparatuses, devices.

## Patentansprüche

1. Mizellenaddukt eines neutralen Komplexes von einwertigem Silber der Formel Ag-L, wobei Ag ein Ag⁺-Ion ist und L ein Ligand der Formel 4-Mercaptophenylboronsäure ist und wobei das Silberion mit der Mercaptogruppe des Liganden L verknüpft ist, in einem Mizellensystem, gebildet durch ein kationisches Tensid.

2. Mizellenaddukt nach Anspruch 1, wobei das kationische Tensid ein quaternäres Ammoniumsalz ist.

3. Mizellenaddukt nach Anspruch 1, wobei das kationische Tensid ausgewählt ist aus der Gruppe bestehend aus Benzyl-(dodecyl)-dimethylammoniumchlorid, Benzyl-(tetradecyl)-dimethylammoniumchlorid, Benzyl-(hexadecyl)-dimethylammonium-chlorid, Benzalkoniumchlorid, Chlorexidin, Polyhexamethylen-Biguanid und Mischungen davon.

4. Verfahren zur Herstellung des Produkts nach einem der Ansprüche 1 bis 3, umfassend die folgenden Schritte:
- Zugabe stöchiometrischer Mengen von 4-Mercaptophenylboronsäure zu einer wässrigen Lösung eines Silbersalzes, um den Komplex Ag-L nach Anspruch 1 zu bilden,
- Zugabe eines kationischen Tensids, um das Mizellenaddukt nach einem der Ansprüche 2 bis 4 zu bilden.

5. Verfahren nach Anspruch 4, wobei das Silbersalz Silbernitrat ist.

6. Verfahren nach Anspruch 4 oder 5, wobei, wenn ein quarternäres Ammoniumsalz als das Tensid verwendet wird, die Konzentration dieses Tensids in der Wasserlösung etwa 10⁻² M oder höher ist.

7. Verfahren nach Anspruch 4 oder 5, wobei, wenn Chlorexidin oder Polyhexamethylen-Biguanid als das Tensid verwendet werden, die Konzentration dieses Tensids in der wässrigen Lösung etwa 0,6-1% w/v ist.

8. Produkt nach einem der Ansprüche 1 bis 3 zur Verwendung in der Medizin.

9. Produkt nach einem der Ansprüche 1 bis 3 zur Verwendung als antimikrobielle Mittel.

10. Pharmazeutische Zusammensetzung, enthaltend eine antimikrobielle Menge des Produkts nach einem der Ansprüche 1 bis 3 zusammen mit pharmazeutisch annehmbaren Hilfsstoffen.

11. Nicht-therapeutische flüssige Formulierung, die das Produkt nach einem der Ansprüche 1 bis 3 enthält.

12. Nicht-therapeutische Verwendung des Produkts nach einem der Ansprüche 1 bis 3 als ein Desinfektionsmittel.

13. Nicht-therapeutische Verwendung nach Anspruch 12 zur Behandlung von Oberflächen von Gegenständen.

14. Nicht-therapeutische Verwendung nach Anspruch 13, wobei die Oberflächen Böden, Wände, Möbel, Geräte, Vorrichtungen sind.

## Revendications

1. Adduit sous forme micellaire d'un complexe neutre d'argent monovalent de formule Ag-L dans lequel Ag est un ion Ag⁺ et L est un ligand de formule acide 4-mercaptophénylboronique, et dans lequel l'ion d'argent est lié au groupe mercapto du ligand L dans un système de micelle formé par un tensioactif cationique.

2. Adduit sous forme micellaire selon la revendication 1, dans lequel le tensioactif cationique est un sel d'ammonium quaternaire.

3. Adduit sous forme micellaire selon la revendication 1, dans lequel le tensioactif cationique est sélectionné dans le groupe constitué du chlorure de benzyl(dodécyl)diméthylammonium, du chlorure de benzyl-(tétradécyl)diméthylammonium, du chlorure de benzyl(hexadécyl)-diméthylammonium, du chlorure de benzalkonium, de la chlorexidine, d'un polyhexaméthylène biguanide et des mélanges de ceux-ci.

4. Procédé de préparation du produit selon l'une quelconque des revendications 1 à 3, comprenant les étapes suivantes :
- l'ajout de quantités stoechiométriques d'acide 4-mercaptophénylboronique à une solution aqueuse d'un sel d'argent pour former le complexe Ag-L selon la revendication 1,
- l'ajout d'un tensioactif cationique pour former un adduit sous forme micellaire selon une quelconque revendication 2 à 4.

5. Procédé selon la revendication 4, dans lequel le sel d'argent est le nitrate d'argent.

6. Procédé selon la revendication 4 ou 5, dans lequel, si un sel d'ammonium quaternaire est utilisé comme tensioactif, la concentration de ce tensioactif dans la solution aqueuse est environ de 10⁻² M ou plus.

7. Procédé selon la revendication 4 ou 5, dans lequel, si la chlorexidine ou un polyhexaméthylène biguanide est utilisé comme tensioactif, la concentration de ce tensioactif dans la solution aqueuse est environ de 0,6 à 1 % en poids/volume.

8. Produit selon l'une quelconque des revendications 1 à 3 pour son utilisation en médecine.

9. Produit selon l'une quelconque des revendications 1 à 3 pour son utilisation comme agent antimicrobien.

10. Composition pharmaceutique contenant une quantité antimicrobienne du produit selon l'une quelconque des revendications 1 à 3 conjointement avec des excipients pharmaceutiquement acceptables.

11. Formulation liquide non thérapeutique contenant le produit selon l'une quelconque des revendications 1 à 3.

12. Utilisation non thérapeutique du produit selon l'une quelconque des revendications 1 à 3 comme désinfectant.

13. Utilisation non thérapeutique selon la revendication 12, pour le traitement des surfaces d'objets.

14. Utilisation non thérapeutique selon la revendication 13, dans laquelle lesdites surfaces sont des sols, des murs, des meubles, des appareils, des dispositifs.
